# EUROPEAN PATENT SPECIFICATION

(11) **EP 4 577 132 B1**
(45) Date of publication and mention of the grant of the patent: **21.10.2026**
(21) Application number: 23768726.4
(22) Date of filing: 23.08.2023
(51) Int. Cl.: A61B 17/3211, A61B 17/32, A61B 17/00, A61B 90/00

(54) **DE QUERVAIN'S TREATMENT DEVICE**
VORRICHTUNG ZUR BEHANDLUNG VON DER QUERVAIN-SEHNENSCHEIDENENTZÜNDUNG
DISPOSITIF DE TRAITEMENT DE LA TÉNOSYNOVITE DE DE QUERVAIN

(30) Priority: 25.08.2022 US 202263401018 P
(43) Date of publication of application: 02.07.2025
(73) Proprietor: Sonex Health, Inc., Eagan, Minnesota 55121 (US)
(72) Inventor: BARNES, Darryl E., Eagan, Minnesota 55123 (US); SMITH, Jay, Byron, Minnesota 55920 (US); HERRIDGE, David, Mendota Heights, Minnesota 55120 (US)
(74) Representative: IK-IP LTD
(86) International application number: PCT/US2023/030909
(87) International publication number: WO 2024/044237

(56) References cited:
- WO-A2-2010/065707
- US-A1- 2017 042 566
- US-B1- 9 456 837

## Description

### CROSS REFERENCE TO RELATED APPLICATIONS

This application claims the benefit of U.S. Provisional Patent Application Serial No.63/401,018 entitled "THUMB TREATMENT DEVICE" and filed August 25, 2022.

### TECHNICAL FIELD

The present application relates generally to medical devices and methods. More specifically, the present application relates to a device and method for treating De Quervain's tenosynovitis and other soft tissue disorders of the hand, wrist, or other parts of the body.

### BACKGROUND

De Quervain's tenosynovitis is a painful condition affecting the two tendons on the thumb side of the wrist. Chronic overuse, such as repeating a particular hand motion day after day, may irritate the sheath around the tendons. If the sheath becomes irritated, the tendons can thicken and swell. This thickening and swelling restrict the movement of the tendons through the small tunnel that connects them to the base of the thumb. Symptoms of de Quervain's tenosynovitis include pain and/or swelling near the base of the thumb, difficulty moving the thumb and wrist when grasping or pinching, and a sticking sensation in the thumb when moving it. In some cases, pain spreads farther into the thumb or forearm.

If conservative treatments for de Quervain's tenosynovitis do not work, surgery is a therapeutic option. In the surgical procedure, the surgeon cuts the sheath around the tendons to release the pressure and allow the tendons to glide freely. As with a trigger thumb release procedure, it is imperative, when treating de Quervain's, to avoid injuring nerve tissue and blood vessels in and around the area where the sheath is cut.

Trigger finger is another example of an ailment of the hand or wrist that is commonly treated with a release procedure. The first annular ("A1") pulley is a small band of tissue on the palmar side of a person's hand. In some cases, the flexor tendon thickens, and a nodule can get caught on the Al pulley and cause irritation and mechanical symptoms. The flexor tendon can then become locked in place when a person flexes his or her fingers. This condition is commonly referred to as "trigger finger." To treat trigger finger, the Al pulley is typically cut, to release the tendon.

Trigger finger can occur in any of the five fingers. Due to the orientation of the thumb relative to the hand and due to the anatomy of the thumb, treating trigger thumb presents different challenges compared to treating the other four fingers. Specifically, the locations of nerves and muscles in the thumb, relative to the A1 pulley and the flexor pollicis longus tendon, make accessing and cutting the A1 pulley to treat trigger thumb technically difficult. It is crucial to a successful trigger thumb release procedure that nerves and blood vessels of the thumb remain undamaged.

As minimally invasive surgery has evolved, numerous tools have been developed to assist in treating medical conditions that have previously been treated with open and more invasive techniques. It would be advantageous to have less invasive devices and techniques for treating de Quervain's tenosynovitis, trigger thumb, and other soft tissue ailments of the hand, wrist, and other parts of the body. Ideally, such tools and techniques would be minimally invasive or at least less invasive than traditional surgical approaches. It would also be advantageous if such devices and methods helped prevent unwanted injury to surrounding neurovascular tissues.

WO 2010/065707 discloses a handpiece for removably attaching one of a plurality of probes including a housing, an actuation assembly arranged on the housing, a linkage assembly arranged on the housing and adapted to couple motion of the actuation assembly to actuation of the probe and an alignment feature arranged on the housing and adapted to control the orientation of a connected probe. US 2017/042566 discloses an endoscopic surgical device for an endoscopic surgical procedure including a slotted clear cannula with a tubular body having a distal end, a proximate end and a slot extending longitudinally therethrough. A pair of wings near the proximal end of the slotted clear cannula extend outward from the tubular body. The slotted clear cannula may be part of an endoscopic surgical blade assembly in which the cannula is attached to a housing enclosing a blade slidably oriented in the cannula.

### SUMMARY

The invention is defined in the independent claim, with optional features being defined in the dependent claims. In one aspect of the present disclosure, a device for treating de Quervain's tenosynovitis may include a handle, an introducer shaft extending from the handle, and a blade that includes a blade shaft extending along a top surface of the introducer shaft and onto the handle and a blade cutting edge that faces toward the handle. The device may further include a blade slider attached to a proximal portion of the blade shaft, a blade pivot attached to at least one of the proximal portion of the blade shaft or the blade slider, an upper deck along which the blade slider slides in an upper horizontal orientation, and a lower deck along which the blade slider slides in a lower horizontal orientation. The blade pivot is configured to allow the blade slider to rotate in a plane that is perpendicular to a plane of the introducer shaft.

In some embodiments, the device may further include a neurovascular guard disposed over the blade. The neurovascular guard is configured to slide along the handle and the introducer shaft. Sometimes, the neurovascular guard may be made of a material that allows passage of ultrasound waves. For example, the material may be, but is not limited to, Ultem, PEEK, PPS, and other polymers. In some embodiments, when the neurovascular guard is placed in an advanced position, a distal tip of the neurovascular guard is located at or near a distal tip of the introducer shaft, and the blade cutting edge is located between the introducer shaft and the neurovascular guard.

In some embodiments, the device may further include an upper guideway adjacent the upper deck and a lower guideway adjacent the lower deck. In such embodiments, the blade pivot rests in and slides along the upper guideway when the blade slider is on the upper deck and the lower guideway when the blade slider is on the lower deck. The upper deck may extend along only a portion of the handle, forming an opening through which the blade pivot can move between the upper guideway and the lower guideway. The blade slider may be configured to move between three orientations via the blade pivot: a blade center horizontal orientation, in which the blade pivot rests in the upper guideway and the blade slider rests on the upper deck; a blade center vertical orientation, in which the blade pivot rests in the upper guideway and the blade slider is rotated to a more vertical position off of the upper deck; and a blade center offset orientation, in which the blade pivot rests in the lower guideway and the blade slider rests on the lower deck. The blade cutting edge in the blade center offset orientation may extend laterally beyond an edge of the introducer shaft. In general, the blade pivot is configured to allow the blade cutting edge to cut a first tissue in approximately a horizontal plane relative to a plane of the introducer shaft and to cut a second tissue in approximately a vertical plane relative to the plane of the introducer shaft.

In another aspect of the present disclosure, a method for treating de Quervain's tenosynovitis in a wrist of a patient may involve: advancing an introducer shaft of a treatment device under an extensor retinaculum; rotating a blade in an upper guideway of the treatment device to a first orientation that is approximately perpendicular to the introducer shaft, to position a cutting edge of the blade distal to the extensor retinaculum; moving the blade proximally to cause the cutting edge to cut through the extensor retinaculum; moving the blade from the upper guideway to a lower guideway in a second orientation that is approximately horizontal to the introducer shaft, where the cutting edge in the second orientation extends laterally beyond an edge of the introducer shaft; and moving the blade proximally to cause the cutting edge to cut through a septum.

In some embodiments, the method may further involve visualizing at least part of the method using an ultrasound device located outside of the patient. Optionally, the method may further involve advancing a neurovascular guard along the introducer shaft before rotating the blade into the first orientation, where the blade is located between the introducer shaft and the neurovascular guard. The method may also further involve advancing the blade along the introducer shaft after advancing the neurovascular guard along the introducer shaft and before rotating the blade into the first orientation.

In some embodiments, the method may involve advancing the blade along the introducer shaft after advancing the introducer shaft and before rotating the blade, where the blade is advanced along the introducer shaft in a third orientation wherein the blade is in the upper guideway and is approximately horizontal to the introducer shaft. The blade may be coupled with a blade slider configured to slide along a handle of the treatment device to slide the blade distally and proximally. The blade may also be coupled with a blade pivot configured to rest in and slide along the upper guideway and the lower guideway. Moving the blade from the upper guideway to the lower guideway may involve moving the blade pivot through an opening in an upper deck of the treatment device to position the blade slider on a lower deck of the treatment device.

The septum that is cut may, in some embodiments, be the first extensor compartment septum. In some embodiments, the septum is cut before the extensor retinaculum is cut. In some embodiments, moving the blade involves sliding and/or rotating a blade slider of the treatment device along a handle of the treatment device, and the blade slider is coupled with a blade shaft of the blade that extends to the cutting edge of the blade.

These and other aspects and embodiments are described in more detail below, in relation to the attached drawing figures.

### BRIEF DESCRIPTION OF THE DRAWINGS

FIGS. 1A and 1B are top views of a trigger thumb treatment device, according to one embodiment;
FIG. 1C is a top view of the trigger thumb treatment device of FIGS. 1A and 1B with a neurovascular guard removed;
FIG. 1D is a top view of the neurovascular guard of the trigger thumb treatment device of FIGS. 1A and 1B;
FIG. 2A is a top perspective view of the neurovascular guard of the trigger thumb treatment device of FIGS. 1A and 1B;
FIG. 2B is a top view (lower panel) and front view (upper panel) of the neurovascular guard of FIG. 2A;
FIG. 2C is a bottom view (lower panel) and rear view (upper panel) of the neurovascular guard of FIG. 2A;
FIGS. 3A and 3B are lateral, cross-sectional views of a thumb and the trigger thumb treatment device, illustrating a method for treating trigger thumb, according to one embodiment;
FIG. 4 is a frontal, cross-sectional view of a portion of a thumb, illustrating a method for treating trigger thumb, according to one embodiment;
FIGS. 5A and 5B are top and side views, respectively, of a neurovascular guard for a trigger thumb or de Quervain's treatment device, according to an alternative embodiment;
FIG. 6 is a perspective view of a treatment device used for treating de Quervain's tenosynovitis, according to one embodiment;
FIG. 7 is a cross-sectional view of a wrist, illustrating placement of a de Quervain's treatment device, according to one embodiment;
FIG. 8A is a perspective view of the de Quervain's treatment device of FIG. 6, with the blade slider and neurovascular guard removed to show additional detail;
FIG. 8B is a posterior view of the de Quervain's treatment device of FIG. 6, showing the blade slider in a blade central horizontal BCH orientation;
FIG. 8C is a posterior view of the de Quervain's treatment device of FIG. 6, showing the blade slider in a blade central vertical BCV orientation;
FIG. 8D is a posterior view of the de Quervain's treatment device of FIG. 6, showing the blade slider in a blade offset horizontal BOH orientation;
FIGS. 9A-9C are top, side, and close-up top views, respectively, of a treatment device used for treating de Quervain's tenosynovitis, according to an alternative embodiment; and
FIG. 10 is a close-up, top view of a distal portion of the treatment device of FIGS. 9A-9C, illustrating one embodiment of a way to move a blade of the device to cut tissue.

### DETAILED DESCRIPTION

The soft tissue cutting devices and methods disclosed in this application are primarily described for use in procedures for treating de Quervain's tenosynovitis and trigger thumb ("trigger thumb release" or "TTR" procedure). The de Quervain's procedure involves cutting the sheath around the two tendons on the thumb side of the wrist. The trigger thumb release procedure involves cutting an A1 pulley in a thumb to release/relieve tension on the flexor pollicis longus tendon. In alternative embodiments, the devices described in this application may be used, or adapted for use, in other parts of the hand, wrist, or body, to cut other soft tissues. For example, the devices and methods described herein may be used (or adapted for use) in treating trigger finger in the other fingers of the hand. Other examples of treatments that may be performed using the methods and devices described in this application include, but are not limited to, cubital tunnel release, extremity compartment release for compartment syndrome, and other fascial tissue releases. Therefore, although this application focuses on de Quervain's and trigger thumb treatments, the devices and methods herein are not limited to those uses. Additionally, any device or method described herein for treating trigger thumb may be used or adapted for use in treating de Quervain's tenosynovitis, and vice versa.

The procedures described herein are typically, but not necessarily, performed under ultrasound guidance, with an ultrasound device positioned outside the hand and/or wrist to visualize structures inside the hand and/or wrist. Additionally or alternatively, an ultrasound transducer may be incorporated into the surgical device itself, so that ultrasound visualization is provided directly inside the hand.

The embodiments of devices and methods described herein are examples only.

In this application, the term "distal" generally means "close to or in a direction toward target tissue," and the term "proximal" generally means "farther from or in a direction away from the target tissue." In other words, proximal and distal are relative terms. For example, when a user holds a treatment device and inserts one end of the treatment device into a patient to perform a treatment, the end of the device that is inserted into the patient will be referred to as the "distal end" of the device. The end of the device being held by the physician will be referred to as the "proximal end" of the device. Although these terms will be used consistently in this application, they should not be interpreted as limiting.

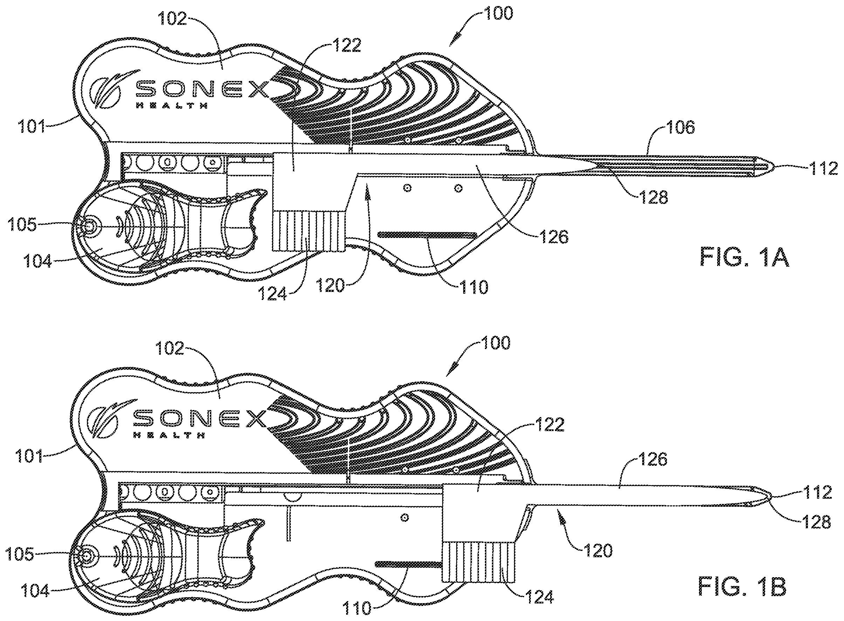

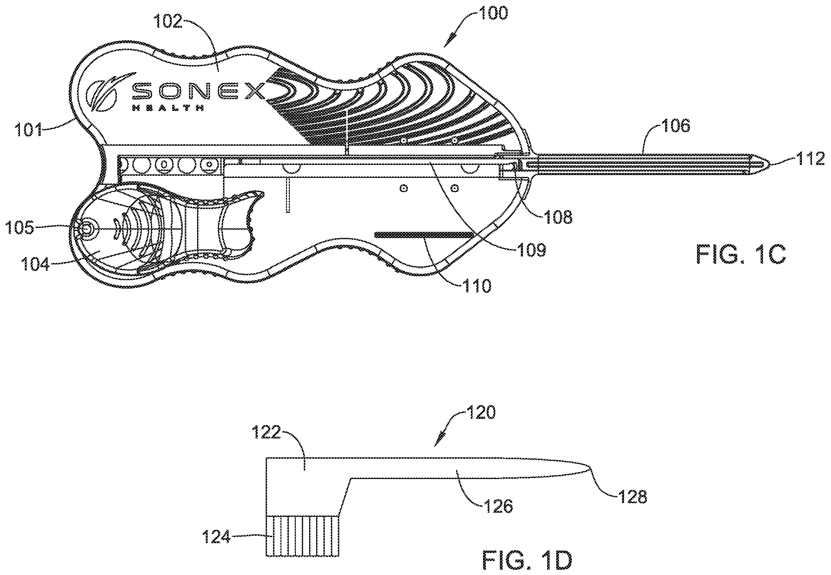

Referring now to FIGS.1A-1D, one embodiment of a thumb treatment device 100 is illustrated. The thumb treatment device 100 may be used, for example, to treat trigger thumb by performing a trigger thumb release ("TTR") procedure. As mentioned above, the thumb treatment device 100 may alternatively be used for other procedures, such as trigger finger release on a finger other the thumb, etc.

In the embodiment of FIGS.1A-1D, the thumb treatment device 100 includes a soft tissue cutting device 101 and a neurovascular guard 120. The soft tissue cutting device 101 is described in detail in U.S. Patent Application No.17/733,532 (Patent No. 12,251,122), entitled "CUTTING DEVICE FOR TRIGGER FINGER AND OTHER SOFT TISSUES," filed April 29, 2022, and U.S. Patent Application No.18/102,990 (Publication No. 2023/240703), entitled "TRIGGER THUMB TREATMENT DEVICES AND METHODS," filed January 30, 2023.

In the illustrated embodiment, the soft tissue cutting device 101 includes a handle 102, a blade slider 104, a post 105 that locks the blade slider 104 in position on the handle 102, an introducer shaft 106 with an introducer tip 112, a blade 108 with a blade shaft 109, and a slot 110 on the handle 102 for accommodating a portion of the neurovascular guard 120. The neurovascular guard 120 includes a guard handle 122, a textured finger slide 124 (the texture enhancing finger gripping ability), a guard shaft 126, and an atraumatic tip 128. When assembled, as in FIGS.1A and 1B, the neurovascular guard 120 fits into the slot 110, for example via snap fit, so that it can slide along the slot 110. The neurovascular guard 120 can be moved from a retracted position (FIG.1A) to an advanced (or "extended") position (FIG.1B). In the advanced position, the atraumatic tip 128 of the neurovascular guard 120 is positioned at or near the introducer tip 112. In use, the neurovascular guard 120 is advanced, then the blade 108 is advanced so its tip is located at or near the introducer tip 112 and the atraumatic tip 128, between the upper surface of the introducer shaft 106 and the lower surface of the guard shaft 126. The blade 108 is then rotated, so the proximally facing cutting surface of the blade 108 is approximately perpendicular (or close to perpendicular), relative to the guard shaft 126 and the introducer shaft 106, with the target tissue to be cut located between the guard shaft 126 and the introducer shaft 106 and proximal of the cutting edge of the blade 108. The slider 104 is then slid proximally along the handle 102 to cause the blade 108 to cut the target tissue, while nearby nerve and vascular tissues are protected from the blade 108 by the guard shaft 126. In some embodiments, the treatment device 100 may include a locking feature to lock the neurovascular guard 120 in the advanced position while performing a tissue cutting procedure. Optionally, the treatment device may also include an additional locking feature to lock the neurovascular guard 120 in a retracted (or most proximal) position, for storage and before the neurovascular guard 120 is deployed.

The neurovascular guard 120 is designed to protect nerve(s) and/or blood vessels in the thumb from the blade 108 during a soft tissue cutting procedure. As nerves in the thumb are typically closer to the area for cutting the A1 pulley than nerves in the other fingers are, the neurovascular guard 120 may be critically important for performing a safe trigger thumb release procedure with the device 100. This may be equally important in cutting the sheath to treat de Quervain's tenosynovitis. In some embodiments, the neurovascular guard 120 is a monolithic structure made of a material that at least partially allows ultrasound waves to pass through it-in other words, the neurovascular guard 120 may be at least partially ultrasound lucent. To achieve this property, the neurovascular guard 120 may be made of any suitable ultrasound lucent material, such as any polymer with physical properties that allow adequate through-transmission or posterior enhancement, such as but not limited to Ultem, PEEK, PPS, or the like. This specific polymer property will allow the user to create an ultrasound image deep to the neurovascular guard 120, making visible the introducer shaft 106 and the blade 108 during placement and transection of the A1 pulley.

In different embodiments, the thumb treatment device 100 may have different lengths for treating different anatomical areas in the thumb (or alternatively other areas of the body). For example, an embodiment of the thumb treatment device 100 used to treat de Quervain's may have several longer portions than another embodiment used to treat trigger thumb. For example, the introducer shaft 106, the blade shaft 109, and the guard shaft 126 may all have differing lengths in different embodiments-e.g., longer for de Quervain's; shorter for trigger thumb.

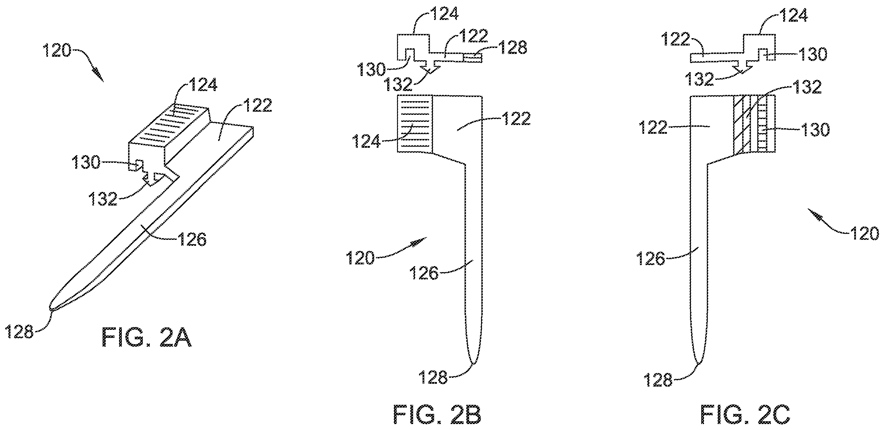

Referring now to FIGS. 2A-2C, the neurovascular guard 120 is illustrated in further detail. FIG. 2A is a perspective view, FIG. 2B is a top view (bottom panel) and front view (top panel), and FIG. 2C is a bottom view (bottom panel) and rear view (top panel). These figures show that the neurovascular guard 120 may include a guard slot 130 and a prong 132. The prong 132 is configured to snap-fit down into the slot 110 on the handle 102 of the device 100. The guard slot 130 is for receiving a longitudinal protrusion on the top surface of the handle 102, which is an optional feature. These features are designed for connecting the neurovascular guard 120 to the handle 102 of the thumb treatment device 100 in a way that allows the neurovascular guard 120 to slide back and forth along the handle 102.

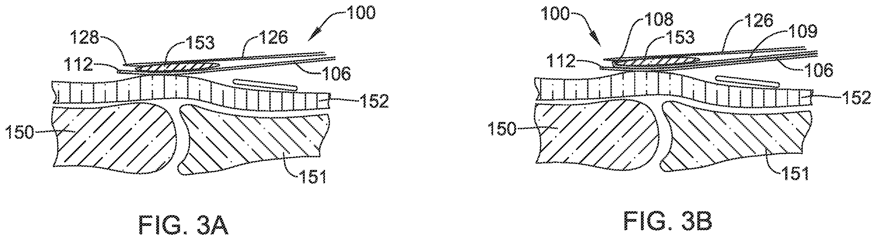

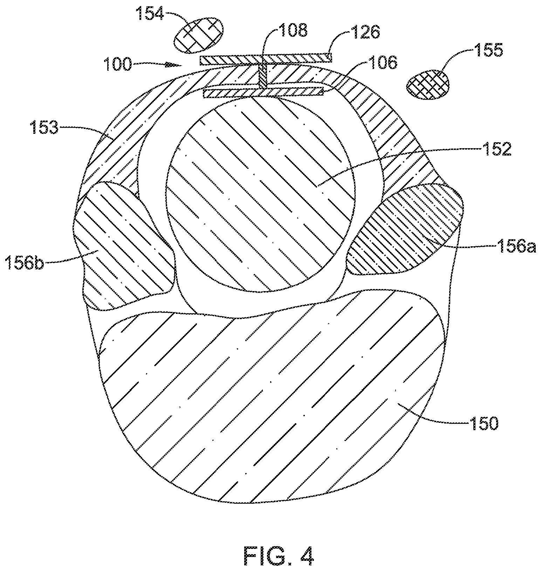

Referring now to FIGS. 3A, 3B and 4, a method for performing a trigger thumb release procedure using the trigger thumb treatment device 100 will be described. First, the anatomy shown in the figures will be described, with FIGS. 3A and 3B being lateral, cross-sectional views, and FIG. 4 being a frontal, cross-sectional view. The anatomical parts shown in FIGS. 3A and 3B are a first metacarpal bone 150, a first proximal phalanx 151, a flexor pollicis longus (FPL) tendon 152, and an A1 pulley 153. FIG. 4 also shows radial and ulnar digital nerve branches 154, 155 and two sesamoid bones 156a, 156b.

Referring to FIG. 3A, in one method embodiment, after surgical preparation with a sterile field and local anesthetic, the thumb is positioned in a slightly extended position with the palm up. Under ultrasound guidance, a small (e.g., 5mm) horizontal incision is made approximately at the mid-point between the interphalangeal (IP) crease and the metacarpal phalangeal (MCP) crease, in a distal to proximal direction, down to the FPL tendon. Then, the introducer tip 112 is used to provide gentle blunt dissection of the region just superficial to the A1 pulley 153. Thereafter, the introducer tip 112 is advanced through the tendon sheath, superficial to the FPL tendon 152, and distally deep to the A1 pulley 153 (as shown in FIG. 3A). The neurovascular guard 120 is unlocked and pushed via the finger slide 124 to the most distal position, so that the guard shaft 126 is superficial to the A1 pulley 153.

Referring to FIG. 3B, the slider 104 (or "blade handle") is then removed from the post 105 and is slid distally along the handle 102 in the horizontal, inactive position (with the blade 108 also horizontal or close to horizontal), to place the blade 108 deep to the A1 pulley 153. The slider 104 is then rotated approximately 90 degrees (counterclockwise), placing the blade 108 in the active (or "vertical" or "perpendicular") position. At this point, the blade 108 is sandwiched between the introducer shaft 106 and the shaft 126 of the neurovascular guide 120, with the blade in a perpendicular (or nearly perpendicular) orientation relative to the two shafts 106, 126. The A1 pulley 153 is similarly sandwiched between the introducer shaft 106 and the shaft 126 of the neurovascular guide 120, and it is located proximally relative to the cutting edge of the blade 108. The slider 104 is then pulled proximally, transecting the A1 pulley 153 with the blade 108, with the neurovascular guide 120 preventing the blade 108 from cutting any of the structures lying above it. After the cut is made, the neurovascular guard 120 is slid proximally via the finger slide 124. The introducer shaft 106 may then be used to confirm complete A1 pulley transection, for example by pulling upward on the handle 102, and is then removed from the patient. If the physician is uncertain that the A1 pulley has been fully transected, the above procedure may be repeated by advancing the neurovascular guard 120 again and repeating the cutting and confirming procedure.

FIG. 4 is an end-on view, showing the introducer shaft 106, the guard shaft 126, and the blade 108, in a position just before cutting the A1 pulley. This view shows the proximity of nerves 154, 155 and thus the importance of the neurovascular guard 120. It also illustrates the orientation of the blade 108 relative to the introducer shaft 106 and the neurovascular guard shaft 126 and the "sandwiching" of the blade 108 between the two shafts 106, 126.

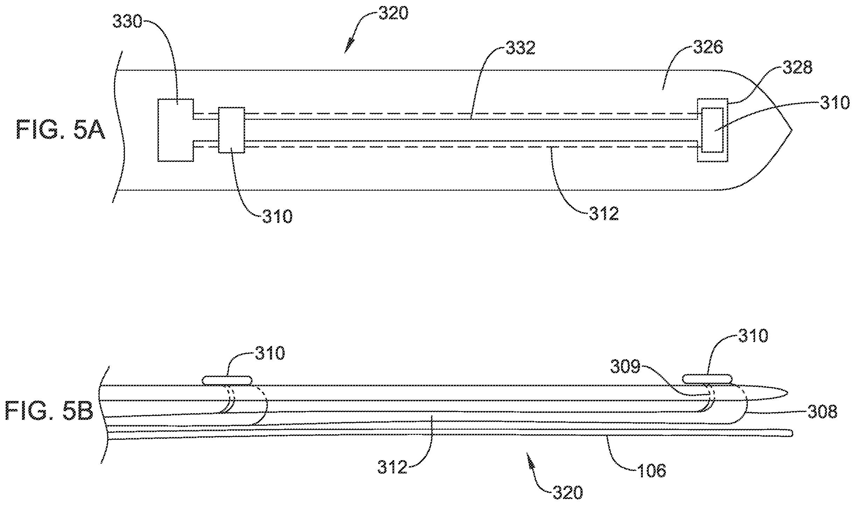

Referring now to FIGS. 5A and 5B, a distal portion of an alternative example of a neurovascular guard 320 is illustrated in top view (FIG. 5A) and side view (FIG. 5B). The proximal portion of the neurovascular guard 320 may be configured the same as, or similar to, the proximal portion of the neurovascular guard 120 pictured in FIGS. 1A-2C. In the embodiment of FIGS. 5A and 5B, the neurovascular guard 320 includes a guard shaft 326 with a slot 332 that has a wide distal opening 328 and a wide proximal opening 330. A different embodiment of a blade 308 may be used with this neurovascular guard 320. This blade 308 includes a cutting edge 309, a blade shaft 312 and a blade capture plateau 310 located at the top of the distal portion of the blade 308. When the blade 308 and the neurovascular guard 320 are positioned at or near the distal end of the introducer shaft 106, as shown toward the righthand portions of FIGS. 5A and 5B, the blade 308 may be rotated from the horizontal position to the vertical position, in which the blade capture plateau 310 (now in a horizontal position) is free to pass through the wide distal opening 328. As the blade 308 slides proximally to cut tissue, the blade capture plateau 310 is wider than the slot 332 and thus is "captured" above the top surface of the neurovascular guard 326. FIGS. 5A and 5B both show the blade 308 in a distal position (to the right) and in a more proximal position (to the left), as when the A1 pulley or other target tissue has been cut.

The slot 332 and the blade capture plateau 310 act like a track to keep the blade 308 and the neurovascular guard 320 oriented consistently relative to one another during a tissue cutting procedure. This embodiment of the neurovascular guard 320 is merely one example, and the slot 332, wide distal opening 328, and wide proximal opening 330 are optional features. In alternative embodiments, the slot 332, wide distal opening 328, and wide proximal opening 330 may be included on the neurovascular guard 120 described above or on other embodiments of neurovascular guards.

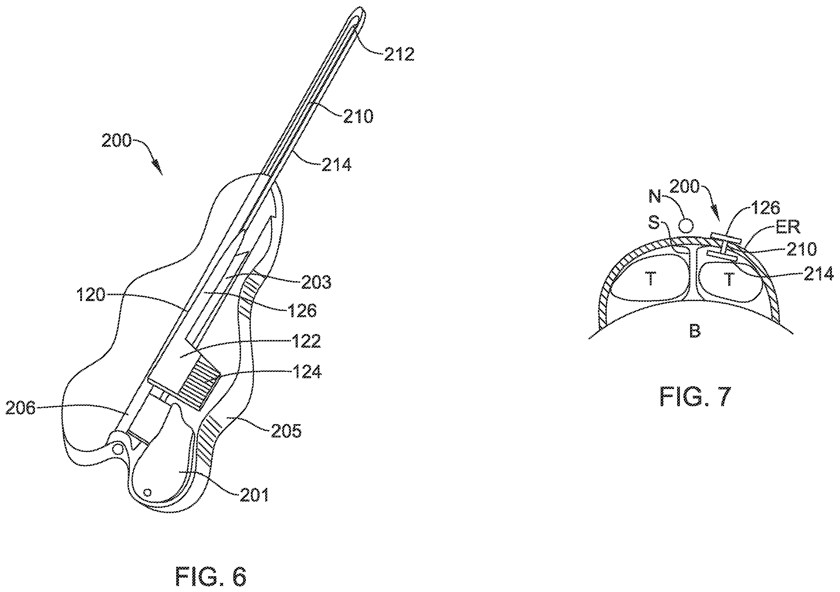

Referring to FIG. 6, one embodiment of a de Quervain's treatment device 200 (hereafter simply "treatment device 200") is illustrated. In treating de Quervain's tenosynovitis surgically, it is often necessary to cut a septum of the sheath and another portion of the sheath. The septum is oriented somewhat vertically, relative to the other portion of the sheath-in other words, the two portions to be cut are approximately perpendicular to one another. For simplicity, this description will refer to the septum orientation as vertical and the other sheath portion orientation as horizontal. This is for description purposes only, however, as either of these two tissue portions may have any of a number of orientations in various embodiments. In general, the two portions simply have different orientations relative to one another.

FIG. 6 is a perspective view of the treatment device 200, showing an introducer handle 205, a blade slider 201, an upper deck 203, an upper guideway 206, a blade shaft 210, a blade 212, and an introducer shaft 214. The treatment device 200 also includes the neurovascular guard 120 described previously, which may have a different size for this treatment device 200. For example, the guard shaft 126 may be longer that in the version used with the treatment device 100, to match a longer introducer shaft 214 of the treatment device 200. The neurovascular guard 120 is an optional feature and may be included in some embodiments and not others. The structure and operation of the treatment device 200 are similar to those of the treatment device 100, except that the blade shaft 210 and blade 212 are able to rotate into two different levels of the treatment device 200, one for cutting in a first orientation (e.g., a "vertical orientation") and the other for cutting in a second orientation (e.g., a "horizontal orientation").

FIG. 7 is a cross-sectional view of a portion of the wrist showing the treatment device 200 in position to cut an extensor retinaculum ER to treat de Quervain's tenosynovitis. Other anatomical structures shown are bone B, tendons T, septum S, and nerve N. In some de Quervain's procedures, the treatment goal is to cut the extensor retinaculum ER and the septum S. Using the treatment device 200, the physician may move the blade shaft 210 and blade 212 to two different orientations-a more vertical orientation for cutting the extensor retinaculum ER and a more horizontal orientation for cutting the septum S. This utility is described in more detail immediately below.

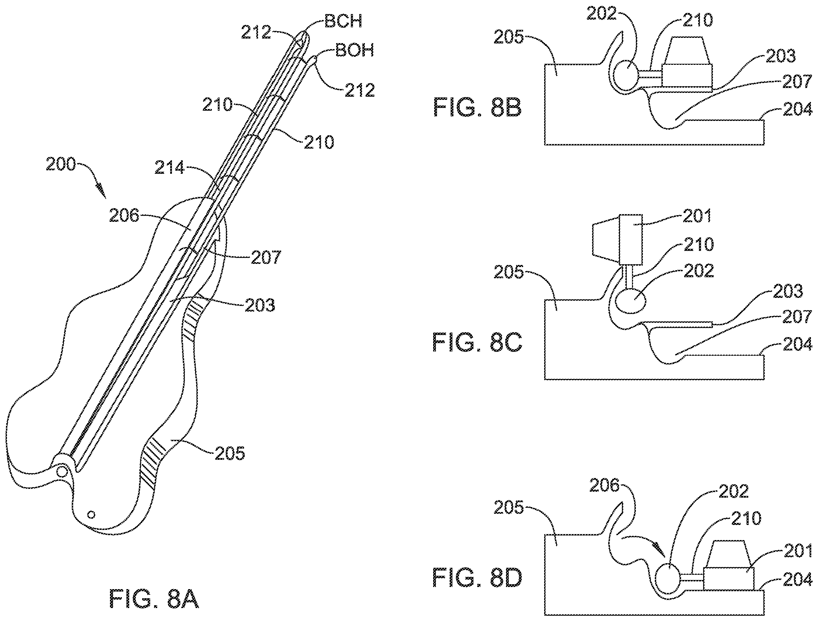

FIG. 8A is a perspective view of the de Quervain's treatment device 200, with the blade slider 201 and the neurovascular guard 120 removed to show further detail of the treatment device 200. FIGS. **8B-8D** are posterior, cross-sectional views of the treatment device 200, showing the blade slider 201 (and thus the blade shaft 210 and the blade 212, which are not visible in these figures) in three different orientations (or "positions") for performing different stages of a de Quervain's treatment procedure. Similarly, FIG. 8A shows the blade shaft 210 and the blade 212 in two different orientations-blade center horizontal BCH and blade offset horizontal BOH. FIGS. 8B-8D show how the blade slider 201 and blade shaft 210 are moved from one orientation to another. FIG. 8B shows the blade slider 201 in the blade center horizontal BCH orientation. FIG. 8C shows the blade slider 201 in another orientation, referred to as the blade center vertical BCV orientation. And FIG. 8D shows the blade slider 201 in the blade offset horizontal BOH orientation. These three orientations and their functions are described immediately below.

Referring to FIG. 8A, the blade shaft 210, which is attached to the blade slider 201 at one end and extends to the blade 212 at the opposite end, can be moved from a blade center horizontal BCH position, where the blade slider 201 rests on an upper deck 203, to a blade offset horizontal BOH position, where the blade slider 201 rests on a lower deck. The upper deck 203 extends along only a proximal portion of the introducer handle 205 of the treatment device 200. Referring to FIG. 8B, with the blade slider 201 resting on the upper deck 203 and the blade shaft 210 in the blade center horizontal BCH position, the blade slider 201 can be advanced along the introducer handle 205 to advance the blade 212 to a location at or near the distal end of the introducer shaft 214. In the blade center horizontal BCH position, as illustrated in FIG. 8D, the blade slider 201 rests on the upper deck 203 and is connected to the blade shaft 210, which in turn is connected to a blade pivot 202. The blade pivot 202 rests in and slides along an upper guideway 206. Thus, the blade center horizontal BCH position is used for advancing the blade 212 to a distal position, in preparation for starting a treatment. When a de Quervain's treatment procedure is complete, the blade 212 may be returned to the blade center horizontal BCH position and either left at or near the distal end of the introducer shaft 214 or retracted to a more proximal location by sliding the blade slider 201 proximally along the upper deck 203. In other words, the blade center horizontal BCH position or orientation may be thought of as an inactive or treatment preparation position.

Once the treatment device 200 is in position for a de Quervain's procedure, the physician may choose to cut the extensor retinaculum first and then cut the septum, to cut the septum first and then extensor retinaculum, or to cut only the extensor retinaculum. Although one example is described here, any combination and order of cuts of any suitable target tissue(s) may be performed using the treatment device 200.

In one example, referring to FIG. 8C, the blade slider 201 may be rotated to a more vertical orientation, the blade center horizontal BCV orientation, by flipping the blade slider 201 upward, causing the blade pivot 202 to rotate in the upper guideway 206. The blade slider 201 may then be slid proximally along the introducer handle 205, causing the proximally facing cutting edge of the blade 212 to cut the extensor retinaculum. If the physician believes the extensor retinaculum is not sufficiently cut on a first past of the blade 212, the blade slider 201 may be advanced and retracted to cut additional tissue in the blade center vertical BCV orientation.

Referring now to FIGS. 8A and 8D, after the extensor retinaculum has been cut (or before it has been cut in some examples), the blade shaft 210 may be moved to the blade offset horizontal BOH position, where the blade slider 201 rests on a lower deck 204 of the introducer handle 205 and the blade pivot 202 rests in a lower guideway 207. The blade slider 201 may be moved to this position by sliding the blade pivot 202 down from the upper guideway 206 to the lower guideway 207 in the area of the introducer handle 205 just distal to the upper deck 203. Again, the upper deck 203 separates the upper guideway 206 from the lower guideway 207 but only extends along a portion of the introducer handle 205, thus leaving room toward the distal end of the introducer handle for the blade pivot 202 to move up or down between the two guideways 206, 207.

In one example of a procedure using the de Quervain's treatment device 200, the physician may first advance the introducer shaft 214 through a small incision, under ultrasound guidance, to position the introducer shaft 214 below (or "deep to") the extensor retinaculum (or other target tissue in other examples). When the introducer shaft 214 is in a desired position, which again may be confirmed using ultrasound visualization, in embodiments that include the neurovascular guard 120, the neurovascular guard 120 may be advanced along the introducer handle 205, to position a distal tip of the guard shaft 126 at or near the distal tip of the introducer shaft 214 and above the extensor retinaculum. The blade slider 201 is then advanced distally in the blade center horizontal BCH orientation (FIG. 8B), with the blade pivot 202 in the upper guideway 206, until the blade 212 is located below and distal to the target tissue. In this position, the blade 212 is located between the introducer shaft 214 and the neurovascular guard shaft 126. The blade slider 201 is then rotated from the blade center horizontal BCH orientation to the blade center vertical BCV orientation (FIG. 8C), thus orienting the cutting edge of the blade 212 approximately perpendicular to and just beyond (distal to) the target tissue (extensor retinaculum). At this point, the blade 212 and the extensor retinaculum are sandwiched between the introducer shaft 214 and the guard shaft 126, with the cutting edge of the blade 212 just distal to the extensor retinaculum that is to be cut.

Next, with the blade slider 201 still in the blade center vertical BCV orientation and the blade pivot 202 in the upper guideway 206, the physician slides the blade slider 201 proximally along the introducer handle 205 to cut through the extensor retinaculum. If the physician wants to then cut a more vertically oriented tissue-e.g., oriented at approximately a 90-degree angle relative to the introducer shaft 214-such as the first extensor compartment septum, the blade slider 201 may be flipped back to the blade center horizontal BCH orientation. The blade slider 201 may then be advanced distally, past the distal end of the upper deck 203, and then laterally and downward, to move the blade pivot 202 from the upper guideway 206 to the lower guideway 207. The blade slider 201, blade shaft 210, and blade 212 are now in the blade offset horizontal BOH orientation (FIGS. 8A and 8D). In this orientation, the cutting edge of the blade 212 extends laterally beyond one edge of the introducer shaft 214, so that when the blade 212 slides proximally, it can cut through tissue just lateral of the introducer shaft 214. In fact, that is the next step in the cutting process. When the physician determines, again possibly via ultrasound guidance, that the blade 212 is in a desired position to cut a target tissue, such as the first extensor compartment septum, the blade slider 201 in the blade offset horizontal BOH orientation, on the lower deck 204, is slid proximally to transect the septum. After that cut is completed, the blade slider 201 may be moved back to the upper deck 203, with the blade pivot 202 in the upper guideway 206, and retracted to a proximal, inactive position. The neurovascular guard 120 may be left in an advanced position or pulled back to a more proximal position. The introducer shaft 214 may then be removed from the patient's wrist. As mentioned above, in alternative examples, the order of tissues cut and/or the target tissues cut may be altered.

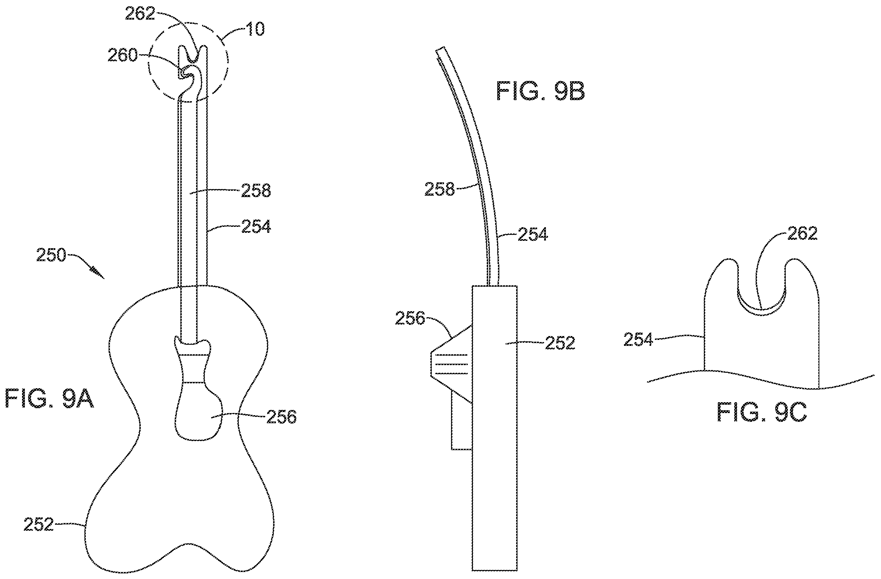

FIGS. 9A-9C are top, side, and front views, respectively, of another embodiment of a treatment device 250 for treating de Quervain's tenosynovitis. In this embodiment, the treatment device 250 includes an introducer handle 252 coupled with an introducer shaft 254 that has a forward cutting blade 262 on its distal end. The treatment device 250 also includes a blade slider 256 coupled with the introducer handle 252 and a blade shaft 258, which ends in a hook blade 260 at its distal end. Using this embodiment, the introducer shaft 254 may be introduced into the patient's hand and the forward cutting blade 262 may be pushed through the septum to cut the septum. The blade slider 256 may then be flipped up to turn the hook blade 260 up into a more vertical position, relative to the introducer shaft 254. The blade slider 256 may then be retracted to cause the hook blade 260 to cut the extensor retinaculum (or other tissue as desired). Before extracting the device from the patient, the blade slider 256 may be flipped back down onto the upper surface of the introducer handle to position the hook blade 260 in a flatter, inactive position.

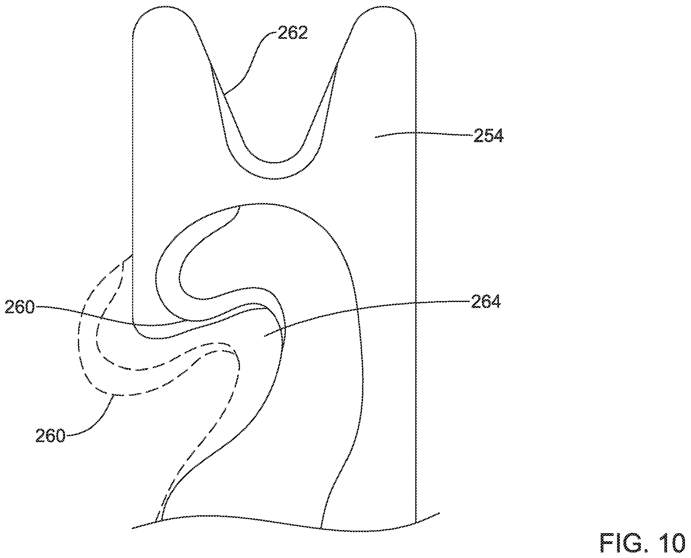

Alternatively, as illustrated in FIG. 10, the blade slider 256 may be pulled back in the flat position, with the hook blade 260 approximately horizontal, to cut tissue through an open area 264 in the introducer shaft 254. In this embodiment, the introducer handle 252 and thus the whole treatment device 250 may be rotated between two different cutting planes during the procedure to cut two different tissues. Also, as illustrated by the dotted-line portion of FIG. 10, the blade slider 256 may be rotated slightly in a lateral orientation relative to the introducer handle 252 to cause the blade 260 to move out laterally from the introducer shaft 254. In this orientation, the blade 260 can cut tissue just lateral of the edge of the introducer shaft 254. Although not described above, the treatment device 250 may optionally include the neurovascular guard 120 or a similar embodiment thereof.

Although the foregoing is believed to be a complete and accurate description of embodiments and features, the invention is not limited to any of the examples or embodiments described herein. The scope of the invention is defined by the claims.

## Claims

1. A device (100, 200) for treating de Quervain's tenosynovitis, the device (100, 200) comprising:
a handle (102, 205);
an introducer shaft (106, 214) extending from the handle (102, 205);
a blade (108, 212), comprising:
a blade shaft (109, 210) extending along a top surface of the introducer shaft (106, 214) and onto the handle (102, 205); and
a blade cutting edge (309) that faces toward the handle (102, 205);
a blade slider (104, 201) attached to a proximal portion of the blade shaft (109, 210);
a blade pivot (202) attached to at least one of the proximal portion of the blade shaft (109, 210) or the blade slider (104, 201), wherein the blade pivot (202) is configured to allow the blade slider (104, 201) to rotate in a plane that is perpendicular to a plane of the introducer shaft (106, 214);
an upper deck (203) along which the blade slider (104, 201) slides in an upper horizontal orientation; and
a lower deck (204) along which the blade slider (104, 201) slides in a lower horizontal orientation.

2. The device of claim 1, further comprising a neurovascular guard disposed over the blade, wherein the neurovascular guard is configured to slide along the handle and the introducer shaft.

3. The device of claim 2, wherein the neurovascular guard is made of a material that allows passage of ultrasound waves.

4. The device of claim 3, wherein the material is selected from the group consisting of Ultem, PEEK, PPS, and other polymers.

5. The device of claim 2, wherein when the neurovascular guard is placed in an advanced position, a distal tip of the neurovascular guard is located at or near a distal tip of the introducer shaft, and wherein the blade cutting edge is located between the introducer shaft and the neurovascular guard.

6. The device of claim 1, further comprising:
an upper guideway adjacent the upper deck; and
a lower guideway adjacent the lower deck,
wherein the blade pivot rests in and slides along the upper guideway when the blade slider is on the upper deck and the lower guideway when the blade slider is on the lower deck.

7. The device of claim 5, wherein the upper deck extends along only a portion of the handle, forming an opening through which the blade pivot can move between the upper guideway and the lower guideway.

8. The device of claim 6, wherein the blade slider is configured to move between three orientations via the blade pivot, the three orientations comprising:
a blade center horizontal orientation, in which the blade pivot rests in the upper guideway and the blade slider rests on the upper deck;
a blade center vertical orientation, in which the blade pivot rests in the upper guideway and the blade slider is rotated to a more vertical position off of the upper deck; and
a blade center offset orientation, in which the blade pivot rests in the lower guideway and the blade slider rests on the lower deck.

9. The device of claim 8, wherein the blade cutting edge in the blade center offset orientation extends laterally beyond an edge of the introducer shaft.

10. The device of claim 1, wherein the blade pivot is configured to allow the blade cutting edge to cut a first tissue in approximately a horizontal plane relative to a plane of the introducer shaft and to cut a second tissue in approximately a vertical plane relative to the plane of the introducer shaft.

## Patentansprüche

1. Vorrichtung (100, 200) zur Behandlung der De-Quervain-Sehnenscheidenentzündung, wobei die Vorrichtung (100, 200) Folgendes umfasst:
einen Handgriff (102, 205);
einen Einführschaft (106, 214), der sich von dem Handgriff (102, 205) aus erstreckt;
ein Messer (108, 212), umfassend:
einen Messerschaft (109, 210), der sich entlang einer oberen Fläche des Einführschafts (106, 214) und auf den Handgriff (102, 205) erstreckt; und
eine Messerschneidkante (309), die dem Handgriff (102, 205) zugewandt ist;
einen Messerschieber (104, 201), der an einem proximalen Abschnitt des Messerschafts (109, 210) befestigt ist;
ein Messerdrehgelenk (202), das an mindestens einem des proximalen Abschnitts des Messerschafts (109, 210) oder des Messerschiebers (104, 201) befestigt ist, wobei das Messerdrehgelenk (202) dazu ausgebildet ist, es dem Messerschieber (104, 201) zu ermöglichen, sich in einer Ebene zu drehen, die rechtwinkelig zu einer Ebene des Einführschafts (106, 214) ist;
ein oberes Deck (203), entlang dessen der Messerschieber (104, 201) in einer oberen horizontalen Ausrichtung verschoben wird; und
ein unteres Deck (204), entlang dessen der Messerschieber (104, 201) in einer unteren horizontalen Ausrichtung verschoben wird.

2. Vorrichtung nach Anspruch 1, weiter umfassend einen neurovaskulären Schutz, der über das Messer angeordnet ist, wobei der neurovaskuläre Schutz dazu ausgebildet ist, entlang des Handgriffs und des Einführschafts verschoben zu werden.

3. Vorrichtung nach Anspruch 2, wobei der neurovaskuläre Schutz aus einem Material gefertigt ist, das den Durchgang von Ultraschallwellen ermöglicht.

4. Vorrichtung nach Anspruch 3, wobei das Material ausgewählt ist aus der Gruppe bestehend aus Ultem, PEEK, PPS und anderen Polymeren.

5. Vorrichtung nach Anspruch 2, wobei, wenn der neurovaskuläre Schutz in einer vorgeschobenen Position angeordnet ist, sich eine distale Spitze des neurovaskulären Schutzes an oder nahe einer distalen Spitze des Einführschafts befindet, und wobei sich die Messerschneidkante zwischen dem Einführschaft und dem neurovaskulären Schutz befindet.

6. Vorrichtung nach Anspruch 1, weiter umfassend:
eine obere Führung neben dem oberen Deck; und
eine untere Führung neben dem unteren Deck,
wobei das Messerdrehgelenk in der oberen Führung liegt und entlang dieser verschoben wird, wenn sich der Messerschieber auf dem oberen Deck befindet, und in der unteren Führung, wenn sich der Messerschieber auf dem unteren Deck befindet.

7. Vorrichtung nach Anspruch 5, wobei sich das obere Deck entlang nur eines Abschnitts des Handgriffs erstreckt und eine Öffnung bildet, durch die sich das Messerdrehgelenk zwischen der oberen Führung und der unteren Führung bewegen kann.

8. Vorrichtung nach Anspruch 6, wobei der Messerschieber dazu ausgebildet ist, sich über das Messerdrehgelenk zwischen drei Ausrichtungen zu bewegen, wobei die drei Ausrichtungen Folgendes umfassen:
eine horizontale Ausrichtung des Messermittelpunkts, bei der das Messerdrehgelenk in der oberen Führung liegt und der Messerschieber auf dem oberen Deck liegt;
eine vertikale Ausrichtung des Messermittelpunkts, bei der das Messerdrehgelenk in der oberen Führung liegt und der Messerschieber an eine vertikalere Position von dem oberen Deck weg gedreht ist; und
eine Ausrichtung mit versetztem Messermittelpunkt, bei der das Messerdrehgelenk in der unteren Führung liegt und der Messerschieber auf dem unteren Deck liegt.

9. Vorrichtung nach Anspruch 8, wobei die Messerschneidkante in der Ausrichtung mit versetztem Messermittelpunkt seitlich über eine Kante des Einführschafts hinausragt.

10. Vorrichtung nach Anspruch 1, wobei das Messerdrehgelenk dazu ausgebildet ist, es der Messeschneidkante zu ermöglichen, ein erstes Gewebe in ungefähr einer horizontalen Ebene relativ zu einer Ebene des Einführschafts zu schneiden, und ein zweites Gewebe in ungefähr einer vertikalen Ebene relativ zu der Ebene des Einführschafts zu schneiden.

## Revendications

1. Dispositif (100, 200) de traitement de la ténosynovite de De Quervain, le dispositif (100, 200) comprenant :
une poignée (102, 205) ;
une tige d'introduction (106, 214) s'étendant depuis la poignée (102, 205) ;
une lame (108, 212), comprenant :
une tige de lame (109, 210) s'étendant le long d'une surface supérieure de la tige d'introduction (106, 214) et sur la poignée (102, 205) ; et
un bord de coupe de lame (309) qui est tourné vers la poignée (102, 205) ;
un curseur de lame (104, 201) fixé à une portion proximale de la tige de lame (109, 210) ;
un pivot de lame (202) fixé à au moins l'un parmi la portion proximale de la tige de lame (109, 210) ou le curseur de lame (104, 201), dans lequel le pivot de lame (202) est configuré pour permettre au curseur de lame (104, 201) de tourner dans un plan qui est perpendiculaire à un plan de la tige d'introduction (106, 214) ;
un plateau supérieur (203) le long duquel le curseur de lame (104, 201) coulisse dans une orientation horizontale supérieure ; et
un plateau inférieur (204) le long duquel le curseur de lame (104, 201) coulisse dans une orientation horizontale inférieure.

2. Dispositif selon la revendication 1, comprenant en outre un protecteur neurovasculaire disposé au-dessus de la lame, dans lequel le protecteur neurovasculaire est configuré pour coulisser le long de la poignée et de la tige d'introduction.

3. Dispositif selon la revendication 2, dans lequel le protecteur neurovasculaire est constitué d'un matériau qui permet le passage d'ondes ultrasonores.

4. Dispositif selon la revendication 3, dans lequel le matériau est sélectionné parmi le groupe consistant en Ultem, PEEK, PPS et d'autres polymères.

5. Dispositif selon la revendication 2, dans lequel, lorsque le protecteur neurovasculaire est placé dans une position avancée, une extrémité distale du protecteur neurovasculaire est située au niveau de l'extrémité distale de la tige d'introduction ou à proximité de celle-ci, et dans lequel le bord de coupe de lame est situé entre la tige d'introduction et le protecteur neurovasculaire.

6. Dispositif selon la revendication 1, comprenant en outre :
une voie de guidage supérieure adjacente au plateau supérieur ; et
une voie de guidage inférieure adjacente au plateau inférieur,
dans lequel le pivot de lame repose dans la voie de guidage supérieure et coulisse le long de celle-ci lorsque le curseur de lame est sur le plateau supérieur, et dans la voie de guidage inférieure lorsque le curseur de lame est sur le plateau inférieur.

7. Dispositif selon la revendication 5, dans lequel le plateau supérieur s'étend le long d'une portion seulement de la poignée, formant une ouverture à travers laquelle le pivot de lame peut se déplacer entre la voie de guidage supérieure et la voie de guidage inférieure.

8. Dispositif selon la revendication 6, dans lequel le curseur de lame est configuré pour se déplacer entre trois orientations via le pivot de lame, les trois orientations comprenant :
une orientation horizontale centrale de lame, dans laquelle le pivot de lame repose dans la voie de guidage supérieure et le curseur de lame repose sur le plateau supérieur ;
une orientation verticale centrale de lame, dans laquelle le pivot de lame repose dans la voie de guidage supérieure et le curseur de lame est tourné vers une position plus verticale hors du plateau supérieur ; et
une orientation centrale décalée de lame, dans laquelle le pivot de lame repose dans la voie de guidage inférieure et le curseur de lame repose sur le plateau inférieur.

9. Dispositif selon la revendication 8, dans lequel le bord de coupe de lame dans l'orientation centrale décalée de lame s'étend latéralement au-delà d'un bord de la tige d'introduction.

10. Dispositif selon la revendication 1, dans lequel le pivot de lame est configuré pour permettre au bord de coupe de lame de couper un premier tissu dans un plan approximativement horizontal par rapport à un plan de la tige d'introduction et de couper un deuxième tissu dans un plan approximativement vertical par rapport au plan de la tige d'introduction.
